# EUROPEAN PATENT APPLICATION

(11) **EP 3 165 524 A1**
(43) Date of publication of application: **10.05.2017**
(21) Application number: 15814736.3
(22) Date of filing: 25.06.2015
(51) Int. Cl.: C07D 285/08, C09B 29/048

(54) **NOVEL AZO COMPOUND AND AZO DYE**

(30) Priority: 03.07.2014 JP 2014138001
(71) Applicant: Fujifilm Corporation, Minato-ku Tokyo 106-8620 (JP)
(72) Inventor: ISHIWATA, Yasuhiro, Ashigarakami-gun Kanagawa 258-8577 (JP); TSUKASE, Masaaki, Ashigarakami-gun Kanagawa 258-8577 (JP)
(74) Representative: Klunker . Schmitt-Nilson . Hirsch
(86) International application number: PCT/JP2015/068424
(87) International publication number: WO 2016/002638

(57) **Abstract**

The present disclosure provides a compound represented by the following formula (1).

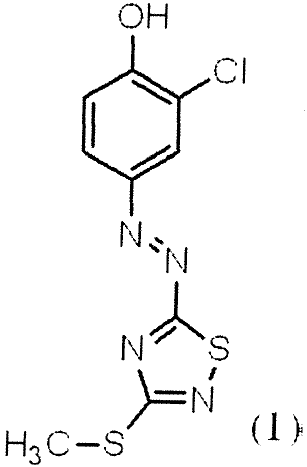

## Description

### BACKGROUND OF THE INVENTION

### 1. Field of the Invention

The present disclosure relates to a novel azo compound and an azo colorant.

### 2. Description of the Related Art

Since many azo colorants have visible light absorption characteristics at various wavelengths, azo colorants have been conventionally utilized as colorants in a variety of fields. For example, azo colorants have been used in a variety of fields such as coloration of synthetic resins, printing inks, colorants for sublimation type thermosensitive image transfer materials, inkjet inks, and colorants for color filters.

A major performance that is required for an azo colorant in connection with colorant functions is the absorption spectrum.

The color of a colorant significantly affects the color tone, texture and the like of an object colored by means of the colorant, and has a significant effect on the visual sense. Therefore, studies concerning the absorption spectra of colorants have been conducted since early times. A general review is described in detail in "Dyes and Pigments", Vol. 3, 123-131 (1982).

In recent years, since color image recording materials have become the mainstream image recording materials, the applications for use of colorants have diversified. Specifically, colorants are popularly utilized in inkjet type recording materials, thermosensitive transfer type recording materials, electrophotography type recording materials, transfer type silver halide photosensitive materials, printing inks, and the like. Furthermore, in regard to photographing equipment, color filters are used in imaging elements such as a charge coupled device (CCD), and in regard to display devices, color filters are used in liquid crystal displays (LCD) or plasma displays (plasma display panels (PDP)), in order to record and reproduce color images.

In these color image recording materials and color filters, coloring agents (dyes and pigments) of three primary colors for a so-called additive color mixing method or subtractive color mixing method are used, in order to reproduce or record full color images.

However, in the current situation, a coloring agent that has absorption characteristics capable of realizing a preferable color reproduction range, can withstand various use conditions and environmental conditions, and has a color with satisfactory fastness, is not available, and improvement is strongly desired.

Meanwhile, in regard to magenta-based dissociative azo colorants, colorants that employ phenol in the coupler portion are widely known (for example, JP1992-331954A (JP-H04-331954A)). However, the azo colorant used for this patented invention needs further improvements in view of heat resistance and light resistance. Furthermore, coloring agents that are used for various applications are commonly required to have preferable absorption characteristics concerning color reproduction, and to have high fastness under the environmental conditions in which the coloring agent is used and a high coefficient of molar light absorption.

Azo dyes containing a 5-membered or 6-membered heterocyclic ring as an azo component have been hitherto disclosed (JP1981-55455A (JP-S56-55455A), JP1985-14243A (JP-S60-14243A), JP1999-125888A, and JP2000-280630A); however, none provides satisfactory properties regarding, for example, color, fastness, and molecular absorption coefficient.

### SUMMARY OF THE INVENTION

An object of the present disclosure is to provide a novel azo compound and a novel azo colorant, both of which have satisfactory color and satisfactory fastness to light and heat, and have a high molecular absorption coefficient.

The inventors of the present invention repeatedly conducted thorough investigations, and as a result, the inventors found that a novel azo compound and a novel azo colorant have satisfactory color and exhibit satisfactory fastness to light and heat. Thus, the inventors completed the embodiments of the present invention.

The invention includes the following embodiments.
[1] An azo compound represented by the following formula (1): 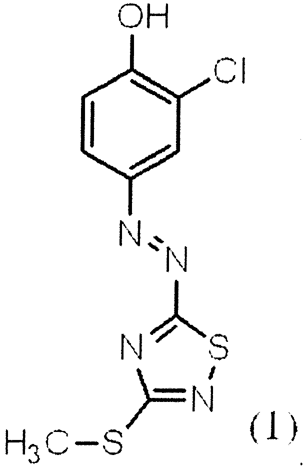
[2] An azo colorant represented by the following formula (1):

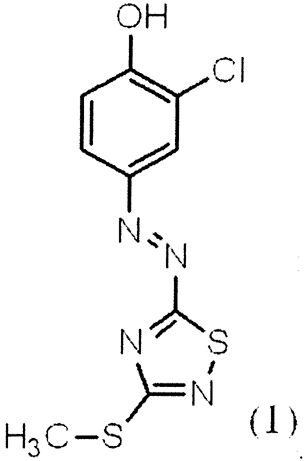

According to the present disclosure, a novel compound and a novel colorant, both of which have excellent color and excellent fastness to light and heat, and have a high molecular absorption coefficient are provided.

### BRIEF DESCRIPTION OF THE DRAWINGS

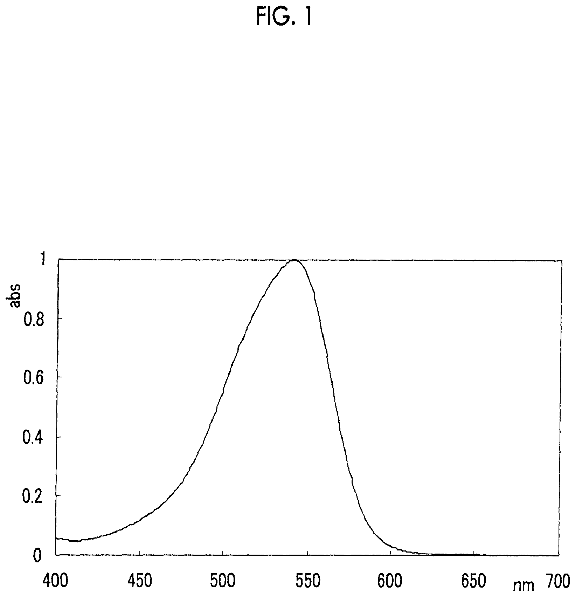

Fig. 1 is the absorption spectrum of the compound synthesized in Example 1 (the solvent is N,N-dimethylformamide).

### DESCRIPTION OF THE PREFERRED EMBODIMENTS

Hereinafter, embodiments of the invention will be described in detail.

According to an embodiment of the invention, there is provided an azo compound represented by the following formula (1):

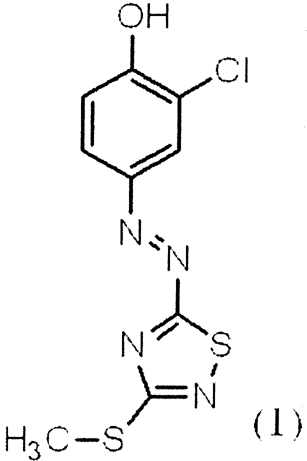

The azo compound represented by formula (1) is 2-chloro-4-((3-(methylthio)-1,2,4-thiadiazol-5-yl)diazenyl)phenol.

The applications for the azo compound represented by formula (1) are not particularly limited; however, it is preferable to use the azo compound as an azo colorant. Examples of the applications for the azo colorant represented by formula (1) include solid-state imaging devices such as a charge coupled device (CCD) and a complementary metal oxide semiconductor (CMOS); color filters for recording and reproducing the color images, which are used in display devices such as liquid crystal displays (LCD) and plasma display panels (PDP); curable compositions for producing color filters; color image recording materials for forming color images; inkjet type recording materials; thermosensitive recording materials; pressure-sensitive recording materials; recording materials using electrophotographic methods; transfer type silver halide photosensitive materials; printing inks; recording pens; and dyes for hair dyeing.

The azo compound and the azo colorant of the present embodiments can be used in a dissolved state, in an emulsified and dispersed state, or in a solid-dispersed state, depending on the system used.

### EXAMPLES

The present embodiments will be described in detail below by way of Examples. However, embodiments of the present invention are not intended to be limited to these Examples.

### Reference Example 1

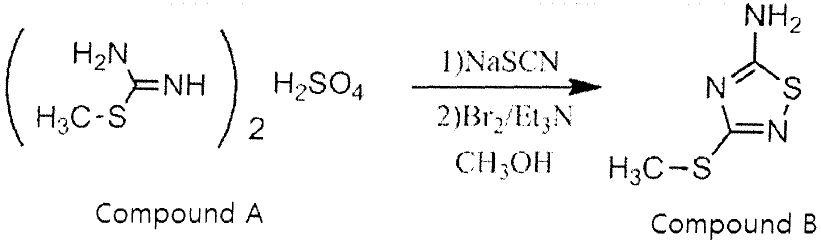

900 mL (milliliters; hereinafter, the same) of methanol was stirred, and 230 mL (1.1 mol) of a 28 mass% methanol solution of sodium methoxide was added thereto. Subsequently, 139 g (0.5 mol) of compound A was added thereto. The internal temperature was adjusted to -5°C using dry ice-methanol, and then 163 g (1.02 mol) of bromine and 205 mL (1.0 mol) of a 28 mass% methanol solution of sodium methoxide were simultaneously added dropwise thereto at an internal temperature of 5°C or lower. After completion of the dropwise addition, the mixture was stirred for 2 hours at an internal temperature of 25°C, and then an inorganic salt thus precipitated was collected by filtration. To the filtrate, an aqueous solution obtained by dissolving 6.4 g of sodium hydrogen sulfite in 35 mL of water was added. Subsequently, the mixture was distilled off under reduced pressure, and a concentrated residue was extracted with water. Thus, 51 g of compound B was obtained (yield: 69.3%).

### Example 1

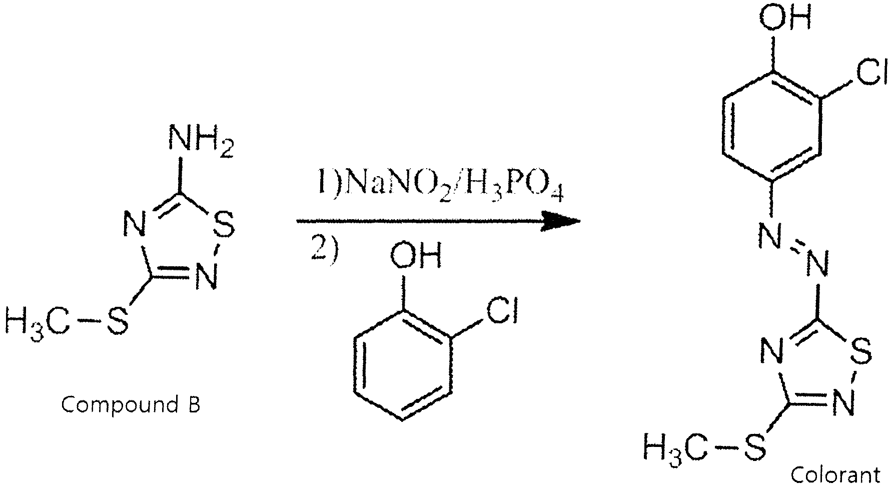

44.2 g (0.3 mol) of compound B obtained in Reference Example 1 was dissolved under heating in 450 mL of a 85 mass% aqueous solution of phosphoric acid, subsequently the internal temperature was maintained to be 5°C or lower in an ice bath, and the solution was subjected to a nitrogen flow. 22.8 g (0.33 mol) of sodium nitrite was added thereto in four divided portions while the internal temperature was maintained at or below 10°C, and a reaction was carried out for 1 hour in the ice bath. Subsequently, a solution obtained by dissolving 38.6 g (0.3 mol) of o-chlorophenol in 400 ml of acetic acid was added dropwise to the reaction mixture while the internal temperature was maintained to be 20°C or lower, and the mixture was stirred for 1 hour at an internal temperature of 20°C. The reaction liquid that had been stirred was added dropwise to 2 L (liters; hereinafter, the same) of water, and crystals precipitated therefrom were collected by filtration and washed with 500 mL of water. The crystals thus obtained were dried and purified by silica gel column chromatography, and the resultant was crystallized out with 15 ml of methanol. Crystals thus precipitated were filtered. Subsequently, the crystals were washed with 1 L of cold methanol and dried, and thus 19.8 g of an exemplary colorant was obtained in the form of brown crystals (yield: 23%).

The exemplary colorant thus obtained had a melting point of 290°C, a maximum absorption wavelength of 540.3 nm, and an absorption coefficient of 60500 (solvent: N,N-dimethylacetamide). This colorant exhibited the absorption spectrum shown in Fig. 1, and had satisfactory color.

For the exemplary colorant obtained as described above, structure identification was implemented according to a nuclear magnetic resonance (NMR) method. The following results of ¹H-NMR (300 MHz, solvent: dimethyl-d⁶ sulfoxide, standard substance: tetramethylsilane) were obtained: [12.2 ppm (1H, s), 8.1 ppm (1H, s), 7.9 ppm (1H, d), 7.2 ppm (1H, d)], [2.8 (3H, s)].

### (Evaluation of fastness)

An evaluation of fastness to light or heat of the compound synthesized in Example 1 was performed by an evaluation method in the same manner as in Example 1 of JP2000-280630A. As a result, the compound synthesized in Example 1 was stable without any decrease in concentration or discoloration observed, and had satisfactory fastness to light or heat.

The entire disclosure of JP2014-138001 is incorporated herein by reference.

All publications, patent applications, and technical standards mentioned in this specification are herein incorporated by reference to the same extent as if each individual publication, patent application, or technical standard was specifically and individually indicated to be incorporated by reference.

## Claims

1. An azo compound represented by the following formula (1):

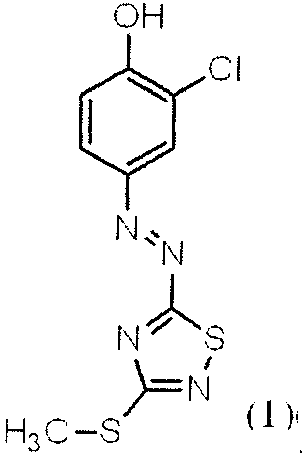

2. An azo colorant represented by the following formula (1):
